(19) Europäisches Patentamt / European Patent Office / Office européen des brevets

(11) **EP 4 455 114 A1**

(12) **EUROPÄISCHE PATENTANMELDUNG**

(43) Veröffentlichungstag:
**30.10.2024 Patentblatt 2024/44**

(21) Anmeldenummer: **23169941.4**

(22) Anmeldetag: **26.04.2023**

(51) Internationale Patentklassifikation (IPC):
**C07C 2/10** (2006.01)      **C07C 11/02** (2006.01)
**C07C 47/02** (2006.01)      **C07C 45/50** (2006.01)

(52) Gemeinsame Patentklassifikation (CPC):
(C-Sets verfügbar)
**C07C 2/10; C07C 45/50;** C07C 2525/02      (Forts.)

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**KH MA MD TN**

(71) Anmelder: **Evonik Oxeno GmbH & Co. KG**
**45772 Marl (DE)**

(72) Erfinder:
• **FRANKE, Robert**
  **45772 Marl (DE)**
• **FRIDAG, Dirk**
  **45721 Haltern am See (DE)**
• **SALE, Anna Chiara**
  **40474 Düsseldorf (DE)**
• **MARKOVIC, Ana**
  **45721 Haltern am See (DE)**
• **PEITZ, Stephan**
  **45739 Oer-Erkenschwick (DE)**
• **GEILEN, Frank**
  **45721 Haltern am See (DE)**
• **KNOSSALLA, Johannes**
  **48351 Everswinkel (DE)**
• **KUCMIERCZYK, Peter**
  **44628 Herne (DE)**
• **BRÄCHER, Alexander**
  **55546 Hackenheim (DE)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

(54) **C8-KOHLENWASSERSTOFFZUSAMMENSETZUNGEN UND EIN VERFAHREN ZU DEREN HERSTELLUNG**

(57) Gegenstand der Erfindung ist eine C8-Kohlenwasserstoffzusammensetzung mit bestimmter Isomerenverteilung sowie ein Verfahren zur Herstellung einer solchen Zusammensetzung mittels Oligomerisierung. Weiterhin betrifft die vorliegende Erfindung die Verwendung der so hergestellten C8-Kohlenwasserstoffzusammensetzung in der Hydroformylierung.

EP 4 455 114 A1

**(Forts. nächste Seite)**

(52) Gemeinsame Patentklassifikation (CPC): (Forts.)

C-Sets
**C07C 2/10, C07C 11/02;**
**C07C 45/50, C07C 47/02**

**Beschreibung**

Die vorliegende Erfindung betrifft eine C8-Kohlenwasserstoffzusammensetzung mit bestimmter Isomerenverteilung sowie ein Verfahren zur Herstellung einer solchen Zusammensetzung mittels Oligomerisierung. Weiterhin betrifft die vorliegende Erfindung die Verwendung der so hergestellten C8-Kohlenwasserstoffzusammensetzung in der Hydroformylierung.

Unter der Oligomerisierung versteht man die Reaktion von Kohlenwasserstoffen mit sich selbst, wobei entsprechend längerkettige Kohlenwasserstoffe entstehen. Oligomerisieren lassen sich insbesondere Olefine (= Alkene), die mindestens eine Kohlenstoff-Doppelbindung im Molekül besitzen. Werden Butene einer Oligomerisierung unterworfen, entstehen dabei im Wesentlichen Olefine mit acht Kohlenstoffatomen (im Folgenden C8-Olefine, sonst auch oft "Dibutene" genannt), Olefine mit zwölf Kohlenstoffatomen (C12-Olefine, "Tributene") sowie in einem geringeren Umfang Olefine mit mehr als zwölf Kohlenstoffatomen (C12+-Olefine).

Die Erfindung befasst sich ausschließlich mit der Oligomerisierung von C4-Olefinen, bei der überwiegend durch Dimerisierung und Trimerisierung C8- und C12-Olefine entstehen.

Zu den C4- Olefinen gehören die vier isomeren Stoffe 1-Buten, cis-2-Buten, trans-2-Buten und Isobuten. 1-Buten und die beiden 2-Butene gehören dabei zur Gruppe der linearen Butene, während Isobuten ein verzweigtes Olefin darstellt. Die linearen C4-Olefine 1-Buten, cis-2-Buten und trans-2-Buten werden auch als "n-Butene" zusammengefasst.

Butene fallen beim Cracken von Erdölfraktionen in einem Steamcracker oder in einem fluidkatalytischen Cracker (FCC) an. Allerdings werden die Butene dabei nicht rein erhalten, sondern als sogenannter "C4-Schnitt". Dies ist ein je nach Herkunft unterschiedlich zusammengesetztes Gemisch aus Kohlenwasserstoffen mit vier Kohlenstoffatomen, welches neben C4-Olefinen auch gesättigte C4-Kohlenwasserstoffe (Butan oder Isobutan) enthält. Butane sind in den meisten industriell verfügbaren C4-Schnitten enthalten und verhalten sich in der Oligomerisierung inert. Des Weiteren können Spuren von Kohlenwasserstoffen mit mehr oder weniger als vier Kohlenstoffatomen (beispielsweise aber nicht ausschließlich Propan und/oder Pentene) und anderen organischen oder anorganischen Begleitstoffen in den C4-Schnitten enthalten sein. Eine alternative Quelle für Butene sind beispielsweise chemische Prozesse, wie die Dehydrierung von Butanen, sowie die fermentative oder die pyrolytische Umwandlung von nachwachsenden Rohstoffen.

Die Herstellung von C8-Olefinen und von C12-Olefinen aus den in C4-Schnitten enthaltenen Butenen ist wirtschaftlich attraktiv und wird deswegen großindustriell betrieben. Die erhaltenen C8-Olefine lassen sich nämlich im Wege der Hydroformylierung mit Kohlenmonoxid und Wasserstoff zu C9-Aldehyden umsetzen, die durch anschließende Hydrierung zu C9-Alkoholen weiterverarbeitet werden. Die C9-Alkohole stellen wiederum einen begehrten Ausgangsstoff für die Herstellung von Weichmachern für PVC dar. In analoger Weise werden die bei der Oligomerisierung von drei Butenen entstehenden C12-Olefine durch Hydroformylierung und Hydrierung zu C13-Alkoholen weiterverarbeitet. Die C13-Alkohole sind ein Vorprodukt für die Herstellung waschaktiver Substanzen.

Eine Oligomerisierung wird in der Regel mehrstufig mit Hilfe einer Reaktorkaskade durchgeführt, die eine der Stufenanzahl entsprechenden Anzahl von hintereinandergeschalteten Reaktionszonen bzw. Reaktoren umfasst. Zwischen den einzelnen Reaktionszonen ist jeweils eine Destillationskolonne vorgesehen, welche die zuvor entstandenen Oligomere aus dem Oligomerisat von den nicht umgesetzten Butenen abtrennt und ausschleust. Die nicht umgesetzten Butene werden teilweise in die vorhergehende Oligomerisierung zurückgeführt und zum anderen Teil der nachfolgenden Oligomerisierung zugeführt.

Durch die Rückführung der nicht umgesetzten Butene herrscht in den ersten Stufen stets ein Überangebot an Butenen, so dass in den einzelnen Oligomerisierungsstufen nie ein vollständiger Umsatz zu C8-, C12- und C12+-Olefinen erreicht wird. Erst eine zwei- bis sechsstufig durchgeführte Oligomerisierung kann insgesamt zu einem nahezu vollständigen Umsatz der eingesetzten Butene führen.

Je nachdem, auf welche Art und Weise sich die einzelnen Buten-Moleküle im Zuge der Oligomerisierung verbinden, erhält man ein Oligomerisat mit unterschiedlichem Verzweigungsgrad. Der Verzweigungsgrad wird durch den Isoindex beschrieben, welcher die mittlere Anzahl der Methylgruppen pro C8-Molekül in dem Isomerengemisch angibt. Der Isoindex für C8-Olefine (Dibuten) ist wie folgt definiert:

$$\text{Isoindex} = (\text{Gewichtsanteil Methylheptene} + 2 \times \text{Gewichtsanteil Dimethylhexene}) / 100$$

So tragen n-Octene mit 0, Methylheptene mit 1 und Dimethylhexene mit 2 zum Isoindex eines Produktgemisches aus C8-Olefinen bei. Je niedriger der Isoindex, umso weniger verzweigt sind die Moleküle innerhalb des Gemisches aufgebaut.

Für die Eigenschaften des Weichmachers spielt nun der Verzweigungsgrad des olefinischen Ausgangsgemisches, das für die Herstellung des Weichmacheralkohols verwendet wird, eine maßgebliche Rolle: Je nach Linearität des C8-Olefingemisches lassen sich die Eigenschaften des daraus hergestellten C9-Weichmacheralkohols einstellen. Bei der Herstellung von Dibuten als Ausgangsprodukt für Weichmacheralkohole wird jedoch oft angestrebt, die Oligomerisierung so zu fahren, dass ein C8-Kohlenwasserstoffzusammensetzung erhalten wird, dessen Isoindex möglichst klein ist.

Als Ausgangsgemisch für die Oligomerisierung kann beispielsweise ein "Raffinat II", welches einen hohen Anteil an 1-Buten aufweist, eingesetzt werden. Unter "Raffinat II" wird gemeinhin ein Butan/Buten-Gemisch verstanden, welches aus C4-Schnitten erhalten wird, die aus Steam-Crackern stammen und aus denen bereits Butadien und Isobuten möglichst vollständig abgetrennt wurden. Restmengen an Butadien und Isobuten im ppm-Bereich können im "Raffinat II" jedoch vorhanden sein.

Neben dem Isoindex spielt für die nachfolgende Hydroformylierung der C8-Olefine und damit für die Qualität des finalen Weichmachers eine wesentliche Rolle, an welcher Stelle innerhalb der n-Octene, Methylheptene und Dimethylhexene sich die Doppelbindung befindet. Es geht also um die Doppelbindungsisomerenverteilung jeweils innerhalb der n-Octenfraktion, der Methylheptenfraktion und der Dimethylhexenfraktion.

Neben dem enormen Einfluss der relativen Anteilen von 1-Buten und 2-Butenen auf die C8-Skelettisomeren wurde nun festgestellt, dass auch die Position der Doppelbindungen bei den verschiedenen C8-Skelettisomeren, die bei der Oligomerisierung gebildet werden, für die Effizienz der Hydroformylierung und die Qualität des im Anschluss gebildeten Weichmachers von großer Bedeutung ist. Dies trifft insbesondere für die Umsatzleistung im Cobalt-Verfahren der Hydroformylierung zu, welches aufgrund seiner geringeren Hydroformylierungsaktivät hochverzweigte Dibutene schlechter zu C9-Aldehyden umsetzen kann als linear ausgeprägte Dibutene. Liegt zudem ein hoher Anteil an innenständigen Doppelbindungsisomeren im hochverzweigten Dibuten vor, verringert sich der Umsatz in der Hydroformylierung zusätzlich, da interne Doppelbindungen für den Co-Katalysator schlechter zugänglich sind. Im Cobalt-Hydroformylierungsverfahren bedeutet also ein hoher Anteil an innenständigen Doppelbindungen in den Dimethylhexenen einen deutlichen Umsatzverlust.

Im alternativen Rhodium- Hydroformylierungsverfahren sind grundsätzlich höhere Umsatzleistungen möglich. Dies liegt darin begründet, dass hierbei höhere Anteile von verzweigten Dibutenen hydroformyliert werden können. Dabei können auch höhere Anteile von innenständigen C8-Doppelbindungsisomeren von verzweigten Dibutenen umgesetzt werden. Dies führt allerdings dazu, dass auch höhere Anteile von verzweigten Oxo-Aldehyden mit in iso-Position befindlicher Aldehydfunktion gebildet werden, die in Folge zu einer ungewünschten Erhöhung der Viskosität des gebildeten Weichmachers führt.

Ein hoher Anteil an innenständigen Doppelbindungsisomeren bei den Dibutenen, die in der Oligomerisierung gebildet werden, kann demnach sowohl zu Umsatzverlusten als auch Qualitätsverlusten bei der Synthese von Weichmachern führen, je nach Hydroformylierungsverfahren.

Es war daher Aufgabe der Erfindung, eine C8-Kohlenwasserstoffzusammensetzung bereitzustellen, die sich gleichermaßen für die Verwendung in Rhodium- oder Cobalt-basierten Hydroformylierungsverfahren eignet und die vorgenannten Nachteile nicht aufweist.

Die Aufgabe konnte durch eine Zusammensetzung gemäß Anspruch 1, die nach dem in Anspruch 5 genannten Verfahren hergestellt worden ist, gelöst werden. Eine solche C8-Kohlenwasserstoffzusammensetzung weist einen möglichst hohen Anteil an bestimmten C8-Doppelbindungsisomeren auf, wodurch ein hoher Umsatz bei der Hydroformylierung bei gleichzeitig möglichst geringem Anteil an hochverzweigten C9-Aldehyden erreicht werden kann

Die erfindungsgemäße Zusammensetzung ist eine C8-Kohlenwasserstoffzusammensetzung, die bei der Oligomerisierung eines butenhaltigen C4-Kohlenwasserstoffstroms entsteht und die die folgende, sich zu mindestens 95%, bevorzugt zu mindestens 97% ergänzende Zusammensetzung aufweist:

1-Octen: 0,01 bis 1,0 Gew.-%, vorzugsweise 0,07 bis 0,7 Gew.-%;
2-Octen: 1,0 bis 15,0 Gew.-%, vorzugsweise 3,0 bis 12,5 Gew.-%;
3-Octen und 4 -Octen zusammen: 2,5 bis 18,0 Gew.-%, vorzugsweise 5,0 bis 16,5 Gew.-%;
3-Methyl-hept-2-en und 3-Methyl-hept-3-en zusammen: 13,5 bis 54,0 Gew.-%, vorzugsweise 20 bis 50 Gew.-%;
2-Ethyl-hex-1-en: 0,1 bis 3,0 Gew.-%, vorzugsweise 0,2 bis 2,2 Gew.-%;
5-Methyl-hept-1-en: 0,0 bis 1,8 Gew.-%, vorzugsweise 0,0 bis 1,6 Gew.-%
5-Methyl-hept-2-en: 9,0 bis 22,0 Gew.-%, 10,0 bis 20 Gew.-%;
5-Methyl-hept-3-en: 2,0 bis 23,0 Gew.-%, vorzugsweise 4,0 bis 15 Gew.-%;
3,4-Dimethyl-hex-2-en: 5,0 bis 29,0 Gew.-%, vorzugsweise 7,5 bis 21 Gew.-%;
3,4-Dimethyl-hex-3-en: 1,5 bis 8,0 Gew.-%, vorzugsweise 2,0 bis 7,5 Gew.-%;
2-Ethyl-3-methyl-pent-1-en: 0,05 bis 3,0 Gew.-%, vorzugsweise 0,1 bis 2,4 Gew.-%;
3-Methyl-hept-1-en: 0,0 bis 2,5 Gew.-%, bevorzugt 0,0 bis 2,0 Gew.-%; und
3,4-Dimethyl-hex-1-en: 0,1 bis 3,0 Gew.-%, bevorzugt 0,2 bis 2,5 Gew.-%.

Somit handelt es sich bei den hergestellten C8-Olefinen in der Regel um n-Octene, Methylheptene und Dimethylhexene. Die Zusammensetzung kann weiterhin bis zu 2 Gew.-% Trimethylpentene, d. h. insbesondere 2,4,4-Trimethyl-pent-1-en und 2,4,4-Trimethyl-pent-2-en enthalten. Die Trimethylpentene entstehen bei der Dimerisierung von Isobuten, welches in geringen Mengen in dem zur Herstellung der erfindungsgemäßen Zusammensetzung verwendeten butenhaltigen C4-Kohlenwasserstoffstrom vorhanden sein kann. Weiterhin kann der Strom bis zu 3 Gew.-% Alkane, beispielsweise n-Octan, 3-Methyl-heptan oder 3,4-Dimethyl-hexan, enthalten. Diese können durch Hydrierung, die als Nebenreaktion auftreten kann, entstehen.

Die erfindungsgemäße C8-Kohlenwasserstoffzusammensetzung weist dabei vorzugsweise einen Isoindex zwischen 0,7 und 1,3, bevorzugt zwischen 0,75 und 1,2 und besonders bevorzugt zwischen 0,8 und 1,1 auf. Die Moleküle in der C8-Kohlenwasserstoffzusammensetzung sind demnach recht moderat verzweigt und weisen im Durchschnitt etwas weniger bis etwas mehr als eine Verzweigung pro Molekül auf.

Wie bereits erwähnt, ist neben dem Verzweigungsgrad der C8-Olefine in der hier dargestellten Erfindung die Lage der Doppelbindungen innerhalb der einzelnen Fraktionen (z. B. n-Octene, Methylheptene und Dimethylhexene) entscheidend für die nachfolgende Umsetzung in einem Hydroformylierungsverfahren und in der Folge für die Qualität des entsprechenden Weichmachers. Dabei ist vor allem wichtig, die Anteile der Doppelbindungsisomere in dem oben genannten Konzentrationsbereich zu halten. Dadurch kann das nachgeschaltete Hydroformylierungsverfahren von vornherein so angepasst werden, dass die Qualität des am Ende der Fertigungskette entstehenden Weichmachers ein Optimum erreicht, ohne Umsatzeinbußen aufgrund zu hoher Anteile an innenständigen Doppelbindungen und stark verzweigter Skelettisomere hinnehmen zu müssen.

Überraschenderweise konnte dieses Ziel erreicht werden, wenn die Oligomerisierung bei vergleichsweise hohen Temperaturen und kurzen Verweilzeiten des eingesetzten butenhaltigen C4-Kohlenwasserstoffstrom beim Durchlaufen eines Oligomerisierungsreaktors betrieben wird.

Ein weiterer Gegenstand der vorliegenden Erfindung ist daher ein Verfahren Herstellung einer C8-Kohlenwasserstoffzusammensetzung mit der oben genannten Zusammensetzung und vorzugsweise mit dem oben genannten Isoindex zwischen 0,7 und 1,3, bevorzugt zwischen 0,75 und 1,2 und besonders bevorzugt zwischen 0,8 und 1,1, durch Oligomerisierung eines butenhaltigen C4-Kohlenwasserstoffstroms mit der folgenden, sich zu 100% ergänzenden Zusammensetzung:

1-Buten: 5 bis 45 Gew.-%;
2-Buten: 20 bis 90 Gew.-%;
Isobuten: 0,1 bis 10 Gew.-%;
n-Butan: 5 bis 80 Gew.-%; und
Isobutan: 0,1 bis 80 Gew.-%;

wobei die Oligomerisierung in einer Reaktionszone in Anwesenheit eines heterogenen Nickel-haltigen Oligomerisierungskatalysators erfolgt.

Ausgangsstoff ist der genannte butenhaltige C4-Kohlenwasserstoffstrom, welcher beispielsweise aus einem Steamcracker oder einem fluidkatalytischen Erdöl-Cracker stammt, einer Vorreinigung unterzogen worden sein kann (bspw. Entfernung von N-, S- oder O-haltigen Verunreinigungen durch Absorption) und vorzugsweise als kontinuierlicher Stoffstrom bereitgestellt wird. Bei dem butenhaltigen C4-Kohlenwasserstoffstrom handelt es sich um ein Gemisch aus Kohlenwasserstoffen mit vier Kohlenstoffatomen, der zumindest die C4-Olefine 1-Buten und 2-Buten (cis und trans), die C4-Alkane n-Butan, Isobutan und optional Isobuten enthält. Der butenhaltige C4-Kohlenwasserstoffstrom kann weiterhin geringe Mengen an weiteren Verbindungen enthalten, die produktionsbedingt vorhanden sind. Beispiele dafür sind C3- oder C5-Verbindungen oder Verbindungen, die mehr als eine Doppelbindung oder andere Mehrfachbindungen enthalten (z. B. Butadien oder Acetylene). Solche Verbindungen sind typischerweise im ppm-Bereich oder darunter enthalten.

Der butenhaltige C4-Kohlenwasserstoffstrom wird in eine Reaktionszone, die aus mindestens einem Reaktor, vorzugsweise zwei oder mehr Reaktoren umfasst, geleitet. Sind mehrere Reaktoren vorhanden sind diese vorzugsweise in Reihe geschaltet. Dabei können zwischen den Reaktoren Destillationskolonnen angeordnet sein, um die gebildeten Oligomere zumindest teilweise abzutrennen, während die restlichen noch nicht umgesetzten Butene zum nächsten Reaktor geführt werden.

Bei der in dem oder in den vorhandenen Reaktor(en) vorherrschenden Reaktionstemperatur, vorzugsweise im Bereich von 50 °C bis 200 °C, besonders bevorzugt 60 °C bis 180 °C, kommt es mit einem in dem oder in den Reaktoren der Reaktionszone angeordneten Oligomerisierungskatalysator in Kontakt, sodass zumindest ein Teil der in dem butenhaltigen C4-Kohlenwasserstoffstrom enthaltenen Butene miteinander zu Oligomeren reagieren und in einem Oligomerisat aus der Reaktionszone abgezogen werden. Das Oligomerisat ist ein Gemisch der gebildeten Oligomere, der nicht umgesetzten Butene und der in der Reaktion sich inert verhaltenen Bestandteile des eingesetzten butenhaltigen C4-Kohlenwasserstoffstroms (z. B. die Butane).

Bei dem in den erfindungsgemäßen Verfahren eingesetzten Oligomerisierungskatalysator handelt es sich um einen heterogenen Nickel-haltigen Katalysator. Bevorzugt wird ein Trägerkatalysator eingesetzt umfassend ein Trägermaterial wie beispielsweise Siliciumdioxid, Aluminiumoxid oder Mischungen daraus wie Alumosilikate oder Zeolithe. Die Träger können Schwefel in Form von Sulfat, Sulfid oder anderen Verbindungsarten enthalten. Der Träger kann weiterhin auch Titandioxid umfassen. Geeignete Oligomerisierungskatalysatoren sind bekannt und werden beispielsweise in der DE 43 39 713 A1, in der WO 2001/37989 A2, in der WO 2011/000697 A1 oder in der WO2020157216 A1 beschrieben.

Für die Herstellung der eingesetzten Nickel-Trägerkatalysatoren gibt es verschiedene Wege. Beispielsweise können solche Katalysatoren durch gemeinsames Fällen von Nickelverbindungen und Trägermaterial (nämlich Aluminium- und/oder Siliziumverbindungen), filtrieren und anschließendes Tempern hergestellt werden. Eine weitere Möglichkeit besteht darin, Nickelverbindungen auf ein geeignetes Trägermaterial aufzubringen, beispielsweise durch Imprägnieren oder Aufsprühen und anschließende Kalzinierung. Zur Herstellung der Katalysatoren nach dem Imprägnierverfahren können Nickelverbindungen wie beispielsweise Nickelnitrat, Nickelchlorid oder Aminkomplexe von Nickel eingesetzt werden. Als Trägermaterialien werden bevorzugt kommerziell erhältliche Katalysatorträger eingesetzt.

Ganz besonders bevorzugt werden Trägerkatalysatoren eingesetzt, die im Wesentlichen aus Nickeloxid, Aluminiumoxid und Siliziumoxid bestehen. Vorzugsweise enthalten diese Katalysatoren 10 bis 50 Gew.-% Nickeloxid, insbesondere 15 bis 40 Gew.-% Nickeloxid. Die Gehalte an Aluminiumoxid liegen im Bereich von 5 bis 30 Gew.-%, insbesondere im Bereich von 7 bis 20 Gew.-%. Die Anteile an Siliziumoxid liegen im Bereich von 10 bis 80 Gew-%. Die angegebenen Mengen der Substanzen, Nickeloxid, Aluminiumoxid und Siliziumoxid ergeben immer 100 Gew.-%. Der Fachmann wird die einzelnen Substanzen daher so kombinieren, dass keine davon abweichende Zusammensetzung entsteht. Als weitere Komponenten können die bevorzugt eingesetzten Trägerkatalysatoren 0.1 bis 2 Gew.-% Alkalioxid, Erdalkalioxid, Lanthanoxid oder Oxide der seltenen Erden enthalten. Zusätzlich können Formgebungshilfsmittel enthalten sein.

Makroskopisch wird der erfindungsgemäß verwendete Nickelkatalysator in einer Gestalt eingesetzt, in der er einen geringen Strömungswiderstand bietet. Bevorzugt liegt der Oligomerisierungskatalysator als Granulat oder in Form von Formkörpern wie Pellets, Tabletten, Zylinder, Kugeln, Strangsextrudaten oder Ringen vor.

Bei der in der Reaktionszone durchgeführten erfindungsgemäßen Oligomerisierung werden überwiegend C8-Olefinen gebildet, wobei die C12-Olefine aber das bedeutendste Nebenprodukt darstellen. Olefine mit mehr als zwölf Kohlenstoffatomen werden nur zu einem vergleichsweisen geringen Anteil gebildet und als C12+-Olefine zusammengefasst.

Das aus der Oligomerisierung erhaltene Oligomerisat ist, wie erwähnt, ein Gemisch aus den gebildeten Oligomeren, den nicht umgesetzten Butenen und den in der Reaktion sich inert verhaltenen Bestandteilen des eingesetzten butenhaltigen C4-Kohlenwasserstoffstroms (z. B. Butane). Nach der Oligomerisierung wird dann in aller Regel eine Abtrennung der erfindungsgemäßen C8-Kohlenwasserstoffzusammensetzung von den nicht umgesetzten Butenen und den inerten Bestandteilen erfolgen. Entsprechende Abtrennverfahren sind dem Fachmann bekannt. In einer bevorzugten Ausführungsform der vorliegenden Erfindung erfolgt die Abtrennung der C8-Kohlenwasserstoffzusammensetzung destillativ. Auch die destillative Abtrennung und die Bedingungen sind dem Fachmann grundsätzlich geläufig. Die C8-Kohlenwasserstoffzusammensetzung wird dabei als Schwersieder im Sumpf anfallen, während die nicht umgesetzten Butene und die inerten Bestandteile am Kopf der Destillation anfallen. Der Kopfstrom kann dann zur Oligomerisierung zurückgeführt werden. Unter Umständen werden aus dem Kopfstrom dabei per Purge Leichtsieder oder ein Teil der inerten Bestandteile entfernt, damit sie sich nicht im Verfahren anreichern.

Die C8-Kohlenwasserstoffzusammensetzung hat, wie erwähnt, ein in der nachfolgenden Hydroformylierung vorteilhafte Zusammensetzung. Durch die Hydroformylierung werden C9-Aldehyde gebildet bzw. eine Mischung aus verschiedenen Isomeren. Solche Aldehyde werden zum Beispiel als Synthesevorstufen, zur Erzeugung von Carbonsäuren und als Riechstoffe genutzt. Technisch werden sie oft durch katalytische Hydrierung in die entsprechenden Alkohole überführt, die u. a. als Zwischenprodukte für die Herstellung von Weichmachern (z. B. Herstellung Diisononylphthalat (DINP) oder 1,2-, 1,3-, oder 1,4-Di-isononylcyclohexandicarbonxylat (z. B. DINCH)) und Detergenzien Einsatz finden.

Ein weiterer Gegenstand der vorliegenden Erfindung ist daher ein Verfahren zur Herstellung eines Aldehydgemisches, wobei die erfindungsgemäß hergestellte C8-Kohlenwasserstoffzusammensetzung einer homogen katalysierten Hydroformylierung zugeführt wird.

Bei der Hydroformylierung werden die in der C8-Kohlenwasserstoffzusammensetzung enthaltenen Olefine mit Synthesegas in Gegenwart eines homogen gelösten Katalysatorsystems hydroformyliert. Das molare Verhältnis zwischen Synthesegas und dem Einsatzgemisch sollte zwischen 6:1 und 1:1, vorzugsweise zwischen 3:1 und 1:1, besonders bevorzug zwischen 2:1 und 1:1 liegen. Die Hydroformylierung kann optional in Anwesenheit eines dem Fachmann bekannten Lösemittels durchgeführt werden.

Das bei der Hydroformylierung einsetzbare homogene Katalysatorsystem kann Cobalt (Co) oder Rhodium (Rh), vorzugsweise Rh, und vorzugsweise einem phosphorhaltigen Liganden umfassen. Phosphorhaltige Liganden sind bei Co nicht unbedingt notwendig. Entsprechende Katalysatorsysteme sind dem Fachmann geläufig. In einer besonders bevorzugten Ausführungsform umfasst oder besteht das homogene Katalysatorsystem aus Rh und einem phosphorhaltigen Liganden.

Der phosphorhaltige Ligand für das erfindungsgemäße Katalysatorsystem ist vorzugsweise ein Phosphin (z.

B. TPP (Triphenylphosphin), ein Monophosphit (z. B. Alkanox 240 (Tris(2,4-di-tert-butylphenyl)phosphit) oder ein Bisphosphit (z. B. Biphephos). Es können auch Mischungen von Liganden eingesetzt werden.

Die Temperatur bei der homogen katalysierten Hydroformylierung liegt vorzugsweise im Bereich von 80 bis 250 °C, weiterhin bevorzugt im Bereich von 90 bis 225 °C und besonders bevorzugt im Bereich von 100 bis 210 °C. Der Druck bei der homogen katalysierten Hydroformylierung liegt vorzugsweise im Bereich von 20 bis 350 bar, weiterhin bevorzugt im Bereich von 30 bis 325 bar und besonders bevorzugt im Bereich von 45 bis 300 bar.

Der Druck bei der Hydroformylierung entspricht üblicherweise dem Gesamtgasdruck. Der Gesamtgasdruck meint im Rahmen der vorliegenden Erfindung die Summe der vorliegenden Drücke aller vorhandenen gasförmigen Stoffe, also den Druck der (gesamten) Gasphase. Im vorliegenden Verfahren entspricht dies insbesondere der Summe der Partialdrücke von CO und $H_2$, d. h. der Gesamtgasdruck ist dann der Synthesegasdruck.

Aus der Hydroformylierung wird dann ein Reaktionsprodukt erhalten, welches die Aldehyde und zumindest nicht umgesetzte Olefine enthält. Die weitere Aufarbeitung des Reaktionsaustrags, insbesondere die Abtrennung des Katalysators und die Abtrennung der Aldehyde, ist dem Fachmann ebenfalls geläufig und kann beispielsweise thermischer Trennung und/oder mittels Membrantrennung erfolgen. Thermische Trennung bzw. thermische Trennverfahren im Sinne der vorliegenden Erfindung meint ein Trennverfahren, bei dem die Trennung anhand des Siedepunktes erfolgt, also z. B. die Destillation.

Die vorliegende Erfindung wird nachfolgend anhand von Beispielen erläutert. Diese Beispiele dienen dazu die Erfindung zu illustrieren, sind aber nicht einschränkend zu verstehen.

Beispiel 1 (nicht erfindungsgemäß)

Das nicht erfindungsgemäße Beispiel 1 wurde in Anlehnung an EP 1 772 445 A1 und bei einem Absolutdruck von 3 MPa in der Flüssigphase bei Temperaturen von 100°C durchgeführt. Als Katalysator wurde ein rein Brönsted-acider Katalysator eingesetzt, Als Einsatzstoff wurde ein Kohlenwasserstoffgemisch verwendet, welches die folgende Zusammensetzung aufwies:

1-Buten 19,0 %
2-Buten 48,0 %
n-Butan 33,0 %

Als Katalysator wurde ein Material verwendet, welches gemäß Beispiel 1 der EP 1 772 445 A1 hergestellt und nach Beispiel 2 derselben Veröffentlichung nachbehandelt worden war. Die Reaktion wurde somit an einem Ni-freien Katalysator durchgeführt. Der bei der Reaktion gebildete Produktstrom enthielt ca. 66 Gew.-% C8-Oligomere und zu ca. 20 Gew.-% C12-Oligomere (Rest C12+-Oligomere und Crack-Produkte).

Der Isoindex des gewonnenen C8-Gemisches liegt bei 1,61. Der Isoindex ist somit deutlich zu hoch.

Die Octen-Doppelbindungsisomere wurde mittels Gaschromatographie (GC) 150 m langen unpolaren Säule auf seine Zusammensetzung untersucht. Das GC wurde vorher mit den Reinsubstanzen und ggf. NMR-Analyse kalibriert. Die entsprechende Zusammensetzung ist in Tabelle 1 aufgeführt. Es werden nur die hier beanspruchten C8-Doppelbindungsisomere aufgeführt. Es ist zu erkennen, dass insbesondere die n-Octene und Methylheptene deutlich unter und die stark verzweigten Dimethylhexene deutlich über den hier bevorzugten Werten liegen. Des Weiteren liegt ein signifikanter Rest an C8-Isomeren in Höhe von 41,1 Gew.-% vor, die keinem der hier beanspruchten C8-Doppelbindungsisomere entsprechen. Der Isoindex des gewonnenen C8-Gemisches liegt bei 1,61. Der Isoindex ist somit deutlich zu hoch.

Tabelle 2: Zusammensetzung der C8-Olefine

| C8-Doppelbindungsisomer | Gew.-% |
|---|---|
| 1-Octen | 0,0 |
| 2-Octen | 0,6 |
| 3-Octen und 4-Octen | 0,8 |
| 3-Methyl-hept-2-en und 3-Methyl-hept-3-en | 9,1 |
| 2-Ethyl-hex-1-en | 0,1 |
| 5-Methyl-hept-1-en | 0,0 |
| 5-Methyl-hept-2-en | 3,0 |

(fortgesetzt)

| C8-Doppelbindungsisomer | Gew.-% |
|---|---|
| 5-Methyl-hept-3-en | 1,6 |
| 3,4-Dimethyl-hex-2-en | 10,2 |
| 3,4-Dimethyl-hex-3-en | 3,3 |
| 2-Ethyl-3-methyl-pent-1-en | 0,5 |
| 3-Methyl-hept-1-en | 0,0 |
| 3,4-Dimethyl-hex-1-en | 29,7 |
| Rest | 41,1 |

Beispiel 2 (erfindungsgemäß)

Das erfindungsgemäße Beispiel 2 wurde bei einem Absolutdruck von 3 MPa in der Flüssigphase bei Temperaturen von 100°C in einem Rohrreaktor durchgeführt. Als Einsatzstoff wurde ein Kohlenwasserstoffgemisch verwendet, welches die folgende Zusammensetzung aufwies:

1-Buten 19,0 %
2-Buten 48,0 %
n-Butan 33,0 %

Als Katalysator wurde nun ein heterogener Ni-haltiger Katalysator (NiO-Gehalt: 35 Gew.-%, Trägermaterial Alumosilikat) eingesetzt. Der bei der Reaktion gebildete Produktstrom enthielt ca. 71 Gew.-% C8-Oligomere und zu ca. 24 Gew.-% C12-Oligomere (Rest C12+-Oligomere und Crack-Produkte).
Die Octen-Doppelbindungsisomere wurden wie in Beispiel 1 untersucht. Die entsprechende Zusammensetzung ist in Tabelle 2 aufgeführt. Es werden nur die hier beanspruchten C8-Doppelbindungsisomere aufgeführt. Es konnte eine erfindungsgemäße C8-Kohlenwasserstoffzusammensetzung erhalten werden. Der Isoindex des gewonnenen C8-Gemisches liegt bei 0,88.

Tabelle 2: Zusammensetzung der C8-Olefine

| C8-Doppelbindungsisomer | Gew.-% |
|---|---|
| 1-Octen | 0,3 |
| 2-Octen | 10,7 |
| 3-Octen und 4-Octen | 14,6 |
| 3-Methyl-hept-2-en und 3-Methyl-hept-3-en | 38,1 |
| 2-Ethyl-hex-1-en | 0,5 |
| 5-Methyl-hept-1-en | 0,0 |
| 5-Methyl-hept-2-en | 12,8 |
| 5-Methyl-hept-3-en | 6,5 |
| 3,4-Dimethyl-hex-2-en | 11,0 |
| 3,4-Dimethyl-hex-3-en | 3,6 |
| 2-Ethyl-3-methyl-pent-1-en | 0,5 |
| 3-Methyl-hept-1-en | 0,0 |
| 3,4-Dimethyl-hex-1-en | 1,4 |

Es konnte also gezeigt werden, dass mit dem erfindungsgemäßen Verfahren die gewünschten C8-Kohlenstoffzusammensetzungen hergestellt werden konnten.

**Patentansprüche**

1. C8-Kohlenwasserstoffzusammensetzung, die bei der Oligomerisierung eines butenhaltigen C4-Kohlenwasserstoffstroms entsteht und die die folgende, sich zu mindestens 95%, bevorzugt zu mindestens 97% ergänzende Zusammensetzung aufweist:

   1-Octen: 0,01 bis 1,0 Gew.-%, vorzugsweise 0,07 bis 0,7 Gew.-%;
   2-Octen: 1,0 bis 15,0 Gew.-%, vorzugsweise 3,0 bis 12,5 Gew.-%;
   3-Octen und 4 -Octen zusammen: 2,5 bis 18,0 Gew.-%, vorzugsweise 5,0 bis 16,5 Gew.-%;
   3-Methyl-hept-2-en und 3-Methyl-hept-3-en zusammen: 13,5 bis 54,0 Gew.-%, vorzugsweise 20 bis 50 Gew.-%;
   2-Ethyl-hex-1-en: 0,1 bis 3,0 Gew.-%, vorzugsweise 0,2 bis 2,2 Gew.-%;
   5-Methyl-hept-1-en: 0,0 bis 1,8 Gew.-%, vorzugsweise 0,0 bis 1,6 Gew.-%
   5-Methyl-hept-2-en: 9,0 bis 22,0 Gew.-%, 10,0 bis 20 Gew.-%;
   5-Methyl-hept-3-en: 2,0 bis 23,0 Gew.-%, vorzugsweise 4,0 bis 15 Gew.-%;
   3,4-Dimethyl-hex-2-en: 5,0 bis 29,0 Gew.-%, vorzugsweise 7,5 bis 21 Gew.-%;
   3,4-Dimethyl-hex-3-en: 1,5 bis 8,0 Gew.-%, vorzugsweise 2,0 bis 7,5 Gew.-%;
   2-Ethyl-3-methyl-pent-1-en: 0,05 bis 3,0 Gew.-%, vorzugsweise 0,1 bis 2,4 Gew.-%;
   3-Methyl-hept-1-en: 0,0 bis 2,5 Gew.-%, bevorzugt 0,0 bis 2,0 Gew.-%; und
   3,4-Dimethyl-hex-1-en: 0,1 bis 3,0 Gew.-%, bevorzugt 0,2 bis 2,5 Gew.-%.

2. C8-Kohlenwasserstoffzusammensetzung nach Anspruch 1, wobei der Isoindex zwischen 0,7 und 1,3, bevorzugt zwischen 0,75 und 1,2 und besonders bevorzugt zwischen 0,8 und 1,1 liegt.

3. C8-Kohlenwasserstoffzusammensetzung nach Anspruch 1 oder 2, wobei die C8-Kohlenwasserstoffzusammensetzung bis zu 2 Gew.-% Trimethylpentene enthält.

4. C8-Kohlenwasserstoffzusammensetzung nach einem der Ansprüche 1 bis 3, wobei die die C8-Kohlenwasserstoffzusammensetzung bis zu 3 Gew.-% Alkane enthält.

5. Verfahren zur Herstellung einer C8-Kohlenwasserstoffzusammensetzung nach einem der Ansprüche 1 bis 4, durch Oligomerisierung eines butenhaltigen C4-Kohlenwasserstoffstroms mit der folgenden, sich zu 100% ergänzenden Zusammensetzung:

   1-Buten: 5 bis 45 Gew.-%;
   2-Buten: 20 bis 90 Gew.-%;
   Isobuten: 0,1 bis 10 Gew.-%;
   n-Butan: 5 bis 80 Gew.-%; und
   Isobutan: 0,1 bis 80 Gew.-%;

   wobei die Oligomerisierung in einer Reaktionszone in Anwesenheit eines heterogenen Nickel-haltigen Oligomerisierungskatalysators erfolgt.

6. Verfahren nach Anspruch 5, wobei der heterogene Ni-haltiger Katalysator ein Trägerkatalysator ist, der ein Trägermaterial aus der Gruppe, bestehend aus Siliciumdioxid, Aluminiumoxid oder Mischungen daraus enthält.

7. Verfahren nach Anspruch 6, wobei der heterogene Ni-haltiger Katalysator 10 bis 50 Gew.-% vorzugsweise 15 bis 40 Gew.-% Nickeloxid, 5 bis 30 Gew.-%, vorzugsweise 7 bis 20 Gew.-% Aluminiumoxid und 10 bis 80 Gew-% Siliziumoxid enthält.

8. Verfahren nach Anspruch 5 bis 7, wobei die Reaktionstemperatur bei der Oligomerisierung im Bereich von 50 °C bis 200 °C, besonders bevorzugt 60 °C bis 180 °C liegt.

9. Verfahren nach Anspruch 5 bis 8, wobei aus der Oligomerisierung ein Oligomerisat enthalten wird, das ein Gemisch aus den gebildeten Oligomeren, den nicht umgesetzten Butenen und den in der Reaktion sich inert verhaltenen Bestandteilen des eingesetzten butenhaltigen C4-Kohlenwasserstoffstroms ist, und anschließend eine destillative Abtrennung der C8-Kohlenwasserstoffzusammensetzung erfolgt.

10. Verfahren zur Herstellung eines Aldehydgemisches, wobei die nach dem Verfahren nach einem der Ansprüche 5 bis 8 hergestellte C8-Kohlenwasserstoffzusammensetzung einer homogen katalysierten Hydroformylierung zugeführt wird.

11. Verfahren nach Anspruch 10, wobei das bei der Hydroformylierung eingesetzte homogene Katalysatorsystem Cobalt (Co) oder Rhodium (Rh), vorzugsweise Rh, und vorzugsweise einem phosphorhaltigen Liganden umfasst.

12. Verfahren nach Anspruch 10 oder 11, wobei die Temperatur bei der homogen katalysierten Hydroformylierung im Bereich von 80 bis 250 °C, vorzugsweise im Bereich von 90 bis 225 °C, besonders bevorzugt im Bereich von 100 bis 210 °C liegt.

13. Verfahren nach einem der Ansprüche 10 bis 12, wobei der Druck bei der homogen katalysierten Hydroformylierung im Bereich von 20 bis 350 bar, vorzugsweise im Bereich von 30 bis 325 bar, besonders bevorzugt im Bereich von 45 bis 300 bar liegt.

Europäisches
Patentamt

European
Patent Office

Office européen
des brevets

# EUROPÄISCHER RECHERCHENBERICHT

Nummer der Anmeldung

EP 23 16 9941

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 199 06 518 A1 (OXENO OLEFINCHEMIE GMBH [DE]) 31. August 2000 (2000-08-31) <br> * Seiten 5-6; Tabelle 4 * <br> * Seite 2, Zeilen 29-33 * <br> ----- | 1-13 | INV. <br> C07C2/10 <br> C07C11/02 <br> C07C47/02 <br> C07C45/50 |
| X | EP 0 730 567 B1 (BASF AG [DE]) 28. Oktober 1998 (1998-10-28) | 1-13 | |
| Y | * Beispiele * <br> * Seite 1, Zeilen 5-10 * <br> ----- | 1-4 | |
| Y | EP 2 947 064 A1 (EVONIK DEGUSSA GMBH [DE]) 25. November 2015 (2015-11-25) <br> * Seite 9 * <br> ----- | 1-4 | |
| A | Geilen Frank M.A. ET AL: "Butenes" In: "Ullmann's Encyclopedia of Industrial Chemistry", 31. Januar 2014 (2014-01-31), Wiley-VCH, Weinheim, XP055966657, ISBN: 978-3-527-30673-2 Seiten 1-13, DOI: 10.1002/14356007.a04_483.pub3, <br> * Seite 4, rechte Spalte * <br> * Seite 7; Tabelle 4 * <br> ----- | 1-13 | |

RECHERCHIERTE SACHGEBIETE (IPC)

C07C

Der vorliegende Recherchenbericht wurde für alle Patentansprüche erstellt

| Recherchenort | Abschlußdatum der Recherche | Prüfer |
|---|---|---|
| Den Haag | 5. Oktober 2023 | Kardinal, Siegmar |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus anderen Gründen angeführtes Dokument
......................................................................................
& : Mitglied der gleichen Patentfamilie, übereinstimmendes
   Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 23 16 9941

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-10-2023

| Im Recherchenbericht angeführtes Patentdokument | Datum der Veröffentlichung | Mitglied(er) der Patentfamilie | | Datum der Veröffentlichung |
|---|---|---|---|---|
| DE 19906518 A1 | 31-08-2000 | AR | 022612 A1 | 04-09-2002 |
| | | BR | 0000487 A | 12-09-2000 |
| | | CA | 2298871 A1 | 17-08-2000 |
| | | CN | 1266835 A | 20-09-2000 |
| | | DE | 19906518 A1 | 31-08-2000 |
| | | EP | 1029839 A1 | 23-08-2000 |
| | | ID | 24790 A | 16-08-2000 |
| | | JP | 2000239196 A | 05-09-2000 |
| | | KR | 20000058062 A | 25-09-2000 |
| | | PL | 338467 A1 | 28-08-2000 |
| | | SG | 89317 A1 | 18-06-2002 |
| | | TW | 491827 B | 21-06-2002 |
| | | US | 6433242 B1 | 13-08-2002 |
| | | ZA | 200000733 B | 08-09-2000 |
| EP 0730567 B1 | 28-10-1998 | DE | 4339713 A1 | 24-05-1995 |
| | | EP | 0730567 A1 | 11-09-1996 |
| | | ES | 2122510 T3 | 16-12-1998 |
| | | JP | 3544980 B2 | 21-07-2004 |
| | | JP | H09505618 A | 03-06-1997 |
| | | US | 5849972 A | 15-12-1998 |
| | | WO | 9514647 A1 | 01-06-1995 |
| EP 2947064 A1 | 25-11-2015 | CN | 105198699 A | 30-12-2015 |
| | | DE | 102014209536 A1 | 26-11-2015 |
| | | EP | 2947064 A1 | 25-11-2015 |
| | | ES | 2660843 T3 | 26-03-2018 |
| | | JP | 6509036 B2 | 08-05-2019 |
| | | JP | 2015218174 A | 07-12-2015 |
| | | KR | 20150133660 A | 30-11-2015 |
| | | MY | 177747 A | 23-09-2020 |
| | | PL | 2947064 T3 | 29-06-2018 |
| | | TW | 201609617 A | 16-03-2016 |
| | | US | 2015336861 A1 | 26-11-2015 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4339713 A1
- WO 200137989 A2
- WO 2011000697 A1
- WO 2020157216 A1
- EP 1772445 A1